# EUROPEAN PATENT APPLICATION

(11) **EP 1 604 670 A1**
(43) Date of publication of application: **14.12.2005**
(21) Application number: 04719140.8
(22) Date of filing: 10.03.2004
(51) Int. Cl.: A61K 31/7004, A61P 3/08, A23L 1/29

(54) **SUPPLEMENTAL FOOD FOR RECOVERY FROM HYPOGLYCEMIC SYMPTOMS**

(30) Priority: 11.03.2003 JP 2003065813
(71) Applicant: ARKRAY INC., Kyoto-shi, Kyoto 601-8045 (JP)
(72) Inventor: SASAKI, Takao, Kyoto-shi, Kyoto 601-8045 (JP); YAMADA, Yasuko., Kyoto-shi, Kyoto 601-8045 (JP)
(74) Representative: Jostarndt, Hans-Dieter
(86) International application number: PCT/JP2004/003156
(87) International publication number: WO 2004/080469

(57) **Abstract**

A supplement food which is useful when the diabetic of recuperation at home lapses into low blood glucose condition, and a supplement food for taking in easily, acting quickly to recovering low blood glucose condition, and, moreover, not having durability after recovery. A supplement food having glucose as the principal component, containing glucose in jelly, and contains 10-20g of glucose in a packaging.

## Description

### FIELD OF THE INVENTION

The present invention relates to a supplement food for recovery from hypoglycemia after taking insulin.

### BACKGROUND OF THE INVENTION

Although supplement foods including sugars for diabetics are provided as a product conventionally, each product is designed for maintaining emitting sugar so as to recover low blood glucose condition in asleep, but there is no product for speedy recovery. For example, there are inventions, such as "DIABETIC SUPPLEMENT BAR" of the publication number WO97/38593, and "USE OF COMPLEX CARBOHYDRATE TO DIMINISH HYPOGLYCEMIA IN PATENTS WITH DIABETES MELLITUS" of publication number WO95/24906.

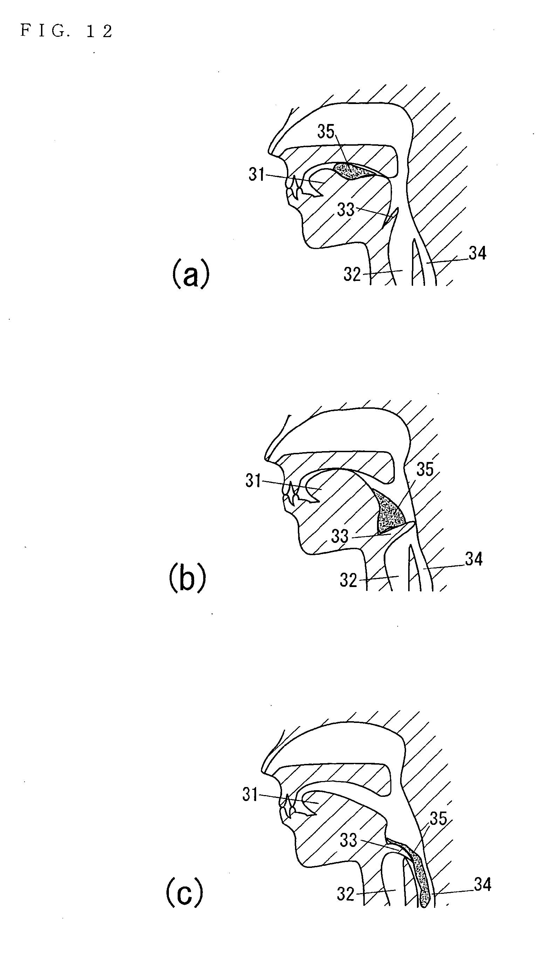

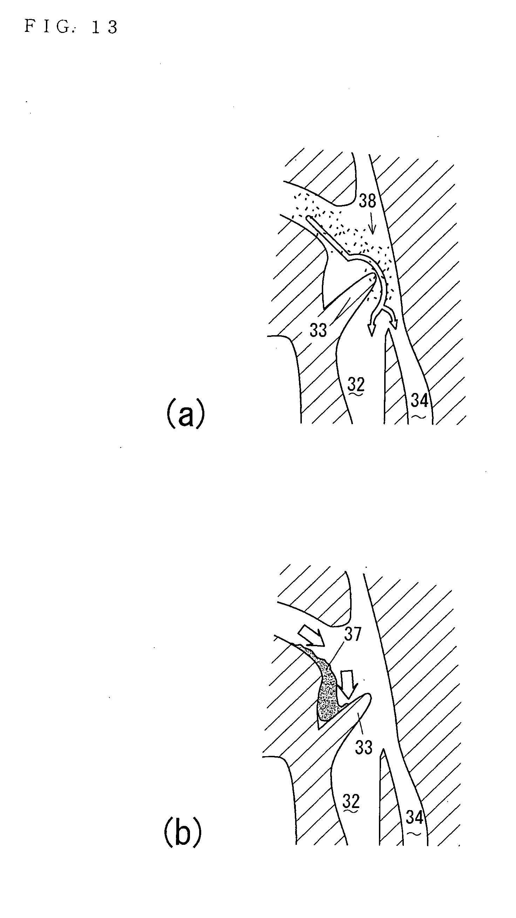

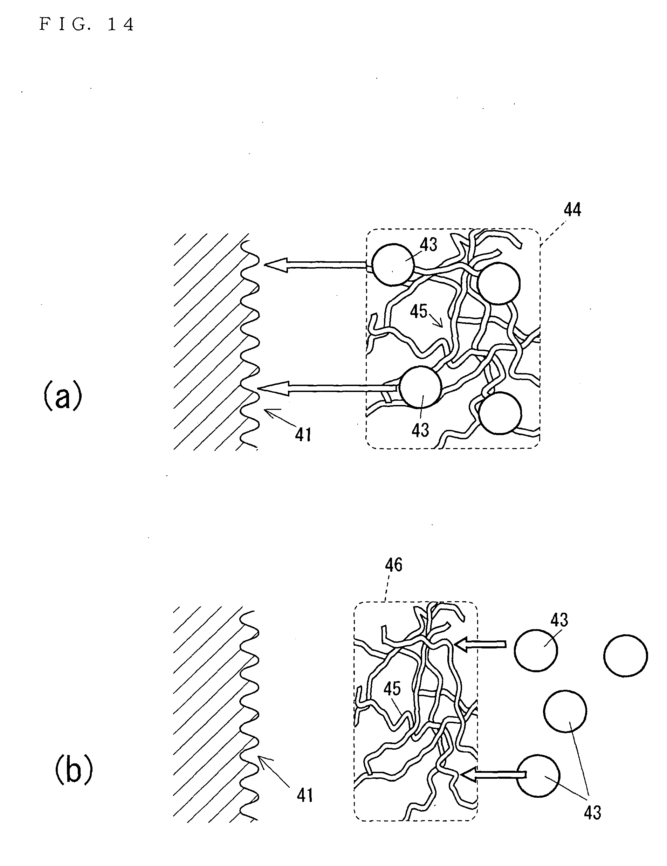

From the former, it is supposed that a supplement food for low blood glucose condition recovery is well-known illustrated in Fig. 12, Fig. 13, and Fig. 14. Fig. 12 is the perspective view of the supplement food of the state of the glucose part package 10, Fig. 13 is the perspective view of the candy-type supplement food 11, and Fig. 14 is the perspective view of the supplement food of tablet-type supplement food 12. However, the pure glucose for recovering low blood glucose condition was supplied from several companies. It is a granule like the glucose part package 10 in Fig. 12, the supplement food 11 like a candy in Fig. 13 or a tablet type supplement food 12 in Fig. 14. Those supplement foods require crunching and swallowing, and it is hard to take in those supplement foods for diabetics who have less function of digestion and saliva secretion. In addition, the stick sugar for coffee or tea is used as supplement food for low blood glucose condition recovery instantly. However, it is dry and powder, it is very hard for the diabetics to take in a stick sugar and the powdered glucose part package 10 of Fig. 12 in low blood glucose condition. Moreover, it is most desirable to take in glucose itself. Since sucrose are made from two sugar, glucose and fructose, and there is also diabetics taking α-glycosidase inhibitor, who cannot disassemble sucrose to fructose and glucose.

With regard to the above-mentioned supplement food for low blood glucose recovery for maintaining emitting sugar, there is fault, for example in the case that a diabetic falls into low blood glucose condition, that if the diabetic ingests a supplement food for low blood glucose recovery for maintaining emitting sugar, the condition of high blood glucose continues after recovering the low blood glucose. Taking a meal in this state causes the control of blood glucose is unsuccessful.

### [Comparison contrast with the supplement food for low blood glucose condition recovery]

The following conventional technology existed.

**[Table 1]**

| | Amount once | Quick-acting | Portability | Easy-opening | Amount of demand | Easy-eating |
|---|---|---|---|---|---|---|
| Powder type glucose (powder part package) | 6 ~ 10 g | ⓞ | ⓞ | ⓞ | ○ | × |
| Tablet type glucose | 2 ~ 5 g | ⓞ | ⓞ | Δ | ○ | Δ |
| stick sugar | 3 ~ 6 g | ○ | ○ | ⓞ | ○ | × |
| Candy type glucose | 3 ~ 5 g | × | ○ | ○ | Δ | ○ |
| Juice type glucose | 350 ml | ○ | × | Δ | Δ | ⓞ |

However, there are no goods which meet all demand at the time of low blood glucose condition.

Next, the fault of the above-mentioned conventional technology will be explained.

Powder type glucose (powder part package) is provided for glycosidase inhibitor users as something extra, and only some, such as a patient and a medical staff, can get it. It is part packaged with a moderate amount, but is hard to swallow since it is powder, although it is a proper quantity, but it is hard to eat with trembling hands at the time of low blood glucose condition.

Tablet type glucose is a solid, makes sweet taste too much, and it is hard to eat without water. Moreover, a blister packed glucose tablet is sealing of every one glucose tablet. It is also hard to eat with trembling hands at the time of low blood glucose condition. And a glucose tablet, which is 2 to 5g, makes a mouth dry and uncomfortable. Although it is necessary to eat two or more pieces at once to take in the glucose of a proper quantity, it is not easy to eat two or more pieces. Candy type glucose is portable, but it is not suitable for urgent case since it takes time to melt. Moreover, there were many sugarless candies etc. and it has the possibility to eating it by mistake. Juice type is not easy to carry about, and the juice containing much glucose is very few. Moreover, it is hard to open package with trembling hands at the time of low blood glucose condition, and drinking juice 100 ml or more is required.

In this invention, the main ingredients may be glucose and fructose liquid made from hydrolysis of saccharose. In the blood glucose in plasma, most is glucose and it is an important energy source for the mammals. By central nervous systems including man's cerebrum, glucose is the only energy source especially. In the time of hungry (5 hour after a meal or subsequent ones), about 8g per hour glucose is emitted from liver, a brain consumes about 1/2 of them, and muscles and erythrocyte consume 1/4, respectively. The blood glucose level is usually adjusted strictly on the balance with the fall by consumption in a muscles, etc. and the rise by production at liver and absorption from intestines. However, if this adjustment is lost, hyperglycemia condition and low blood glucose condition will occur.

It is called diabetes that a hyperglycemia state continues, and this is generated, when the hormone called the insulin secreted from the pancreas stops working by various causes and it becomes impossible for a cell to use the glucose in blood. If a hyperglycemia state continues, it will combine with the protein of the cell which is making the blood vessel, and superfluous glucose will make the function of cell original lose. For this reason, the obstacle of the blood vessel with detailed eye, kidney, and nerve is carried out, and it suffers from retinopathy of diabetes, nephropathy, and neurosis, and when critical, it must become blind, or it must dialyze by renal insufficiency, or a walk becomes difficult. Moreover, it becomes easy to start myocardial infarction by arteriosclerosis. This is a diabetic condition. Now, the number of diabetics is about 6 million, it is said that about 600,000 people need insulin medical treatment, and 60,000 people need insulin medical treatment are sent to hospital, and remaining 540,000 people are sending house medical treatment.

Low blood glucose condition is a condition which the blood glucose level falls and is conversely generated beyond necessity with the diabetic above-mentioned insulin injection and above-mentioned diabetic blood glucose descent medicine of a curative medicine.

Although the usual fiducial point is 70 - 109 mg/dl, as for the blood glucose level, in low blood glucose condition, the state of 50 or less mg/dl serves as a standard. As a cause of raising low blood glucose, much quantity of the medicine, such as insulin, delayed mealtime, less eating, drinking, intense movement, diarrhea, vomiting, the combined use with another medicine, etc. are raised. And it is easy to appear in time at the time of hungry before a meal. If it takes in immediately even if low blood glucose condition appears, sweet thing, i.e., sugar, it will recover soon.

As a low blood glucose condition, it makes Shaking and a feeling chill, Pounding heart, cold Sweating, Hunger, and feeling of exhaustion, headache, an uneasy feeling, nausea, and flitting eyes and impatience and nervousness. Furthermore, when condition becomes heavy, the symptoms of unusual speech and convulsions, a coma, or consciousness dim may be shown. As prevention and treatment of low blood glucose condition, it is important to take in a sweet immediately.

However, the low blood glucose condition makes a situation that one can't think clearly and can't judge coolly. If suitable sugar is not supplied in the situation, one will fall in the tendency to eat anything got hands on. For the reason, the control of blood glucose level after low blood glucose recovery is in disorder, and it serves a situation which is bad for a mental health.

In low blood glucose condition, becoming symptoms of shocking condition and generating of the critical condition by the fall of the energy supply capability to a head are known. Moreover, among the patients who usually have restricted the sweet thing, there are not few patients who think "I may eat a sweet thing if I become low blood glucose". Such a view is strictly mistaken in diabetic medical treatment, and cannot maintain suitable blood glucose control. Guidance taking in sugar at the time of low blood glucose and providing no suitable goods are the reason having such a view.

Also in a diabetic, since the inpatient is undergoing a medical specialist's medical treatment, also when it becomes low blood glucose condition which the glucose level in blood becomes extremely low after insulin injection etc. even if, one can undergo a doctor's medical treatment promptly. However, on the other hand, when the patient of house medical treatment becomes low blood glucose condition, urgent correspondence is needed.

Usually, for the diabetic of house medical treatment in a house, it is also possible to forward mealtime. But in the situation of being decided mealtime such as under a school and service, or in the situation of being hard to take in food such as under a meeting and operation, etc., taking in a stick sugar is required for raising the blood glucose level.

However, sugar is dry powder or a tablet, and it is very hard to take in it. Moreover, although sugar is disaccharide made from two sugars, glucose and fructose, since the patient (taking α-glycosidase inhibitor) who cannot metabolize sugar to fructose and glucose also exists. So, it is most desirable to take in glucose itself.

However, although the pure glucose for recovering low blood glucose condition is supplied from several companies, all have the shape of a granule, and a tablet, and it is very hard to take in for the patients to whom the digestion function is falling, and the patients to whom the saliva secretion function is falling.

The supplement food for low blood glucose condition recovery is required easy to carry and effective with small quantity.

Then, we provide a supplement food which is easy to take in and recovers the low blood glucose condition immediately when fallen into the low blood glucose condition, and which is not durability after recovery.

### SUMMARY OF THE INVENTION

The problem which the invention solves is like the above, and the means for solving is explained.

As specified in claim 1, the present invention features a supplement food for low blood glucose condition recovery which has glucose as the main ingredient of the sugar for low blood glucose recovery at the time of low blood glucose condition, and being jelly-like property.

As specified in claim 2, the supplement food contains glucose 10-20g in a packaging.

As specified in claim 3, the supplement food is made its glucose concentration 20 - 65%.

As specified in claim 4, the supplement food contains 15-50g in a packaging.

As specified in claim 5, the Supplement food is made hardness of the jelly 10 - 100 g/cm² at 20°C.

Since this invention is the supplement food for low blood glucose condition recovery constituted as mentioned above, the following effects are done so.

The 1st, it will be in the state of being easy to take in by making glucose into the shape of jelly even if a lot of sugar does not have water, either. Even if there is no water, a lot of sugar and high-concentration sugar can be taken in easily.

The 2nd, since it is jelly-like, it is easy to take in high-concentration glucose.

The 3rd, intense sweet taste can be eased according to the masking effect of a gelling agent by making sugar into the shape of jelly.

The 4th, since glucose is made into the main ingredients of the sugar for low blood glucose recovery and 10g or more of glucose is included, the blood glucose level rises promptly.

The 5th, since its size is palm and small packing of about 30g, and portable.

The 6th, even if there is no water, a lot of sugar and high-concentration sugar can be taken in easily.

The 7th, since glucose is made into the main ingredients, those it is also useful for who are using α-glycosidase inhibitor

The 8th, the blood glucose level is promptly recoverable by taking in glucose or the sugar of a low molecule at the time of low blood glucose.

### BRIEF EXPLANATION OF THE DRAWINGS

Fig. 1 is a plane view showing the case of the operation of lamination tube packing.
Fig. 2 is a perspective diagram of Fig. 1.
Fig. 3 is a perspective diagram of a recipe state.
Fig. 4 is a perspective diagram showing the case of the operation of the bending opening container 2 that the extraction mouth 2b thereof can be opened only by bending operation.
Fig. 5 is a perspective diagram showing the front side of the bending opening container 2 similarly.
Fig. 6 is a perspective diagram in the state where carried out the opening of the bending opening container 2, and it is similarly taken.
Fig. 7 is a perspective diagram of a case of the operation by which the screw cap 4 was formed in the extraction part although it was lamination packing.
Fig. 8 is a perspective diagram of the container of the model which the small container made of resin and the screw cap 4 combined.
Fig. 9 is a perspective diagram of the small container made of resin, and a bending section type container.
Fig. 10 is a perspective diagram of the tube type container made of aluminum foil or resin.
Fig. 11 is a figure showing the ingestion form of glucose, and blood glucose level change.
Fig. 12 is a figure showing the process which swallows food.
Fig. 13 is a figure showing the inflow process of food in near the epiglottis.
Fig. 14 is a figure showing the absorption state of the gel-like thing containing glucose.

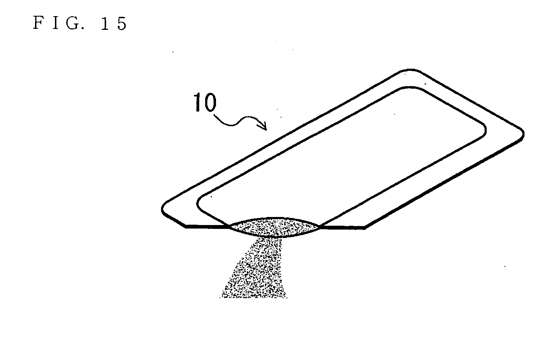

Fig. 15 is a perspective diagram of the auxiliary food for low blood glucose condition recovery of the state of the glucose part package 10.

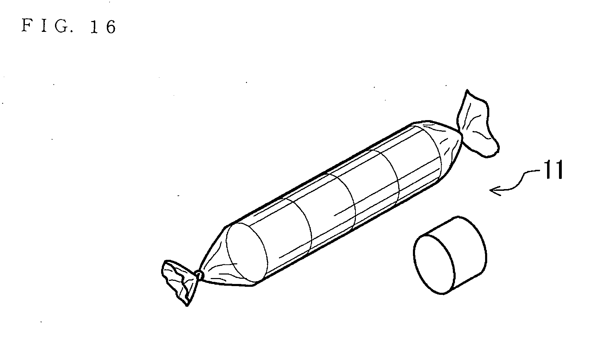

Fig. 16 is a perspective diagram of the auxiliary candy-like food 11 for low blood glucose condition recovery.

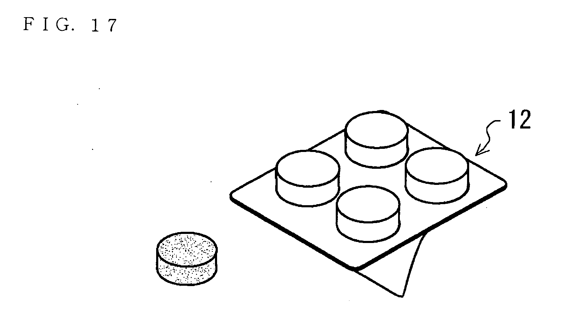

Fig. 17 is a perspective diagram of the auxiliary tablet-like food 12 for low blood glucose condition recovery.

### THE BEST MODE OF THE INVENTING

This invention is what having glucose as the main ingredient for low blood glucose recovery.

Glucose is one of the aldohexose, and D-glucose also called glucose, and is typical aldohexose. Among monosaccharide, with D-fructose, a distribution of glucose is the largest, and it exists so much in sweet fruits in the state of isolation, and is contained a little also in blood, cerebrospinal fluid, and lymph, and exists so much in a diabetic's urine. Moreover, that is in disaccharide (maltose, saccharose, lactose, etc.) or in polysaccharide (cellulose, a starch, glycogen, etc.), and quantity that exists as a composition component of glycoside further, and that is obtained according to these hydrolyses. D-glucose is reducing sugar which has sweet taste that it is easy to melt into water. Glucose cannot melt into an alcohol easily and is insoluble to ether. The annular isomer is known variously and alpha- and beta-D-glycopyranose are one of things with a pyranose ring. It is alpha type with the water of crystallization of one molecule which was crystallized from water, and it is 86 °C of melting point. It is beta type without water of crystallization which was crystallized from ethyl alcohol and methyl alcohol, and it is 146.5 °C of melting point, and specific rotation [α] D=+109.6 degree. beta type will be obtained, if alpha type is heated in pyridine and it crystallizes at 0 degree C, and they are 148-150 °C of melting point, and [specific rotation alpha] D=+20.5 degree. Both molds show mutarotation in an aqueous solution, and an eventual value is [alpha] D=+52.3 degree. A transfer in this balanced state is remarkably promoted by the alkali. Although alpha- and beta-glcofuranose is also one of things with a furanose ring, comparing with a pyranose type, it is unstable and it is ethyl glucoside and a few sorts of the derivative that is obtained in crystalline state. The phenylhydrazone of D-glucose are levorotatory, 160 °C (alpha type) and 141°C (beta type) of melting point, and D-glucosazone are levorotatory, 145 °C (alpha type), and 205 °C (beta type) of melting point. In addition, various glucoside, ether, ester, etc. are known in nature or artificially. Being oxidized makes D-gluconic acid and reduction of D-saccharic acid makes D-sorbitol, vacuum deposition gives alpha-glucosan and beta-glucosan. Glucose is used as an energy source for many of living organism, and it ferments with yeast. Naturally L-glucose does not exist, but it is obtained from L-arabinose from nature. Melting point is 143 °C (alpha type) and specific rotation [α] D=-51.4° (eventual value). The glucosazone is dextrorotatory and cannot melt in water easily, and is 163 °C of melting point. DL-glucose carries out the mixed dissolution of the equivalence of D-glucose and L-glucose, or is artificially compounded by reduction of DL-gluconic acid lactone. DL-glucosazone is 218 °C of melting point.

In this invention, the above-mentioned glucose is constituted so that 10-20g may be included in 1 packaging (Quantity indicated by diabetes medical-treatment guidelines). And glucose concentration to the whole is set up to 20 - 65%. Further, the supplement food for low blood glucose condition recovery of this invention can add a gelatinizer, the charge of flavor, vitamins, and minerals, and it is packaged so that whole capacity may be set to 15-50g (preferably 30g) at 1 packaging. And the supplement food for low blood glucose condition recovery is constituted in the shape of a gel or a jelly like a jam, and chewing and swallowing become easy. The quality of the shape of the jelly does not have resistance to the teeth like a Gummy candy level. It is not necessary to crunch by this and swallowing becomes easy.

For agent of gelatinization for making it the shape of a jelly, Agar, gelatin, pectin, a thickening polysaccharide, lecithin (for example, an egg or the thing of the soybean origin), modificated lecithin (for example, enzyme processing or the thing by which acetylation was carried out), carrageenan, xanthene gum, Monod and diglyseride, guar-gum, carboxymethylcellulose, stearoilchocorate succinic acid monoglyceride, sugar fatty acid ester, diacetil tartaric acid ester of monoglyceride, the poly-glyceloce ester of fatty acid, etc. can be used.

In order to make appearance beautiful, an additive colorant and an acidulant of natural or artificial can be added for the supplement food for low blood glucose condition recovery of this invention. Sweet taste becomes lighter with addition of the acidulant.

Moreover, agent of flavors may be applied to the low blood glucose condition recovery supplement food of this invention to for one's taste.

The flavor can be an extract with strawberries, cherries, orange, vanilla, spices such as nutmeg, cinnamon, and can be ester, a volatile oil, chocolate flavor, a raisin, chocolate, a coconut, and a peanut. Moreover, one or more sorts of a natural or an artificial flavor may be available. The additives of flavor may be an extract and volatile oil. An extract is a pure anise extract, an imitation banana extract, an imitation cherry extract, a chocolate extract, a pure lemon extract, a pure orange extract, a pure peppermint extract, an imitation pineapple extract, an imitation ram extract, an imitation strawberry extract, or a pure vanilla extract, and an volatile oil is a palm oil, bay oil, bergamot oil, a cedar oil, a cherry oil, a cinnamon oil, a clove oil, peppermint oil, or a peppermint oil.

In order to prolong a preservation life, antiseptics can also be added to the supplement food for low blood glucose condition recovery. Preferably, a sorbic acid potassium salt, sorbic acid sodium, benzoic acid potassium, a sodium benzoate, or EDTA calcium sodium, are used as a antiseptics.

Furthermore, it is desirable to add vitamins and minerals to the supplement food for low blood glucose condition recovery. The above-mentioned vitamins here specifically, for example, vitamin A 1, A2 and A3, vitamin B1 (thiamine), B-2 (riboflavine), B6 (pyridoxine), vitamin B12, vitamin C (ascorbic acid), vitamin D 1, D2, vitamin E, Vitamin F, the vitamin H (biotin) vitamin K, Vitamin P and L-carnitine (vitamin B T), Vitamin U, and a niacin (nicotinic acid), also the precursor of vitamins, such as B-carotene may be useful. Moreover, inositol, folic acid, pantothenic acid, and a choline acid may be useful. With the above-mentioned minerals, calcium, Lynn, iodine, iron, magnesium, copper, zinc, manganese, a chloride, potassium, sodium, selenium, chromium, molybdenum, etc. may be useful.

### Case of the operation (prescription)

**[Table 2]**

| component | |
|---|---|
| glucose | 34 g |
| gelatin | 2 |
| agar | 0.05 |
| ascorbic acid | 0.4 |
| sodium ascorbate | 0.2 |
| yoghurt flavor | 0.3 |
| water | 63.5 |
| totaI | 100.45 g |

Ascorbic acid is an acidulant. Ascorbic acid and a sodium ascorbate make a buffer solution.

Glucose, gelatin, and agar were mixed to purification water and it stirred and scattered to it an agitated part, it heated to it for 5 minutes at 85 °C, and the gel component was dissolved in it. There, the yogurt flavor was added there and it mixed. By ascorbic acid and the sodium ascorbate, pH was prepared to 4.0.

As a gel quality, the hardness and viscosity of a jelly were prepared as following so that swallowing might become easy without crunching.

The jelly hardness in 20 °C is desirable 10 - 100 g/cm². It is possible to make miss-deglutition that jelly hardness is less than 10 g/cm². Moreover, if jelly hardness is over 100 g/cm², biting will become difficult for those to whom the capability to bite is falling. When the resistance (N/^{m2}) when compressing at fixed velocity shows the hardness of the food which can be crushed with the gum or a tongue, it is below 5x10⁴ (N/m²) (Japan Health Food & Nutrition Food Association *specification of the food for elderly people).* Moreover, it is supposed that one can swallow, without crunching that hardness is less 5x10³ N/m².

The polyethylene back was filled with the obtained gel-like solution so that the amount of contents might become 30g, and sealed with the desk impulse sealer. At 85 °C, boil pasteurization was carried out for 20 minutes, and water cooling was carried out after that.

Next, the case of the operation of a packaging state of the supplement food for low blood glucose condition recovery of this invention is explained.

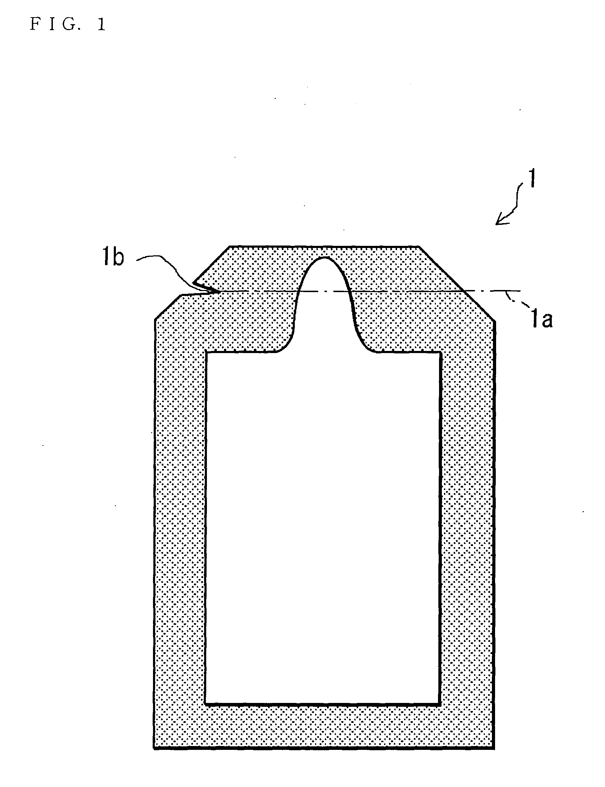

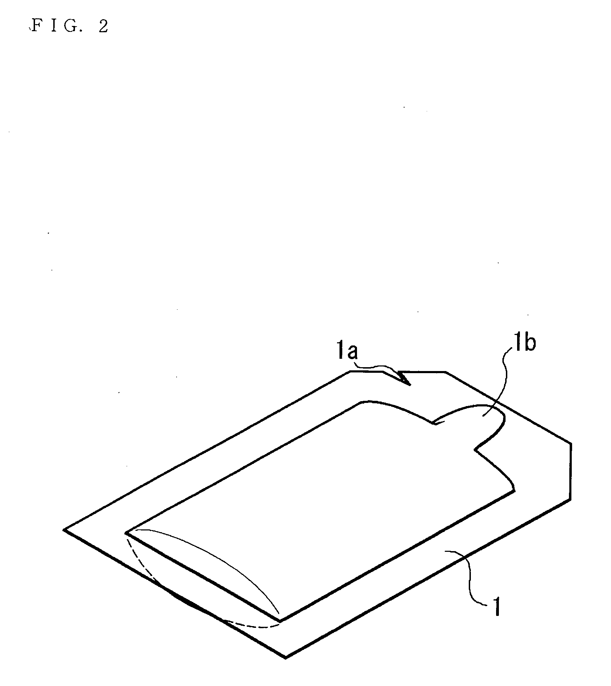

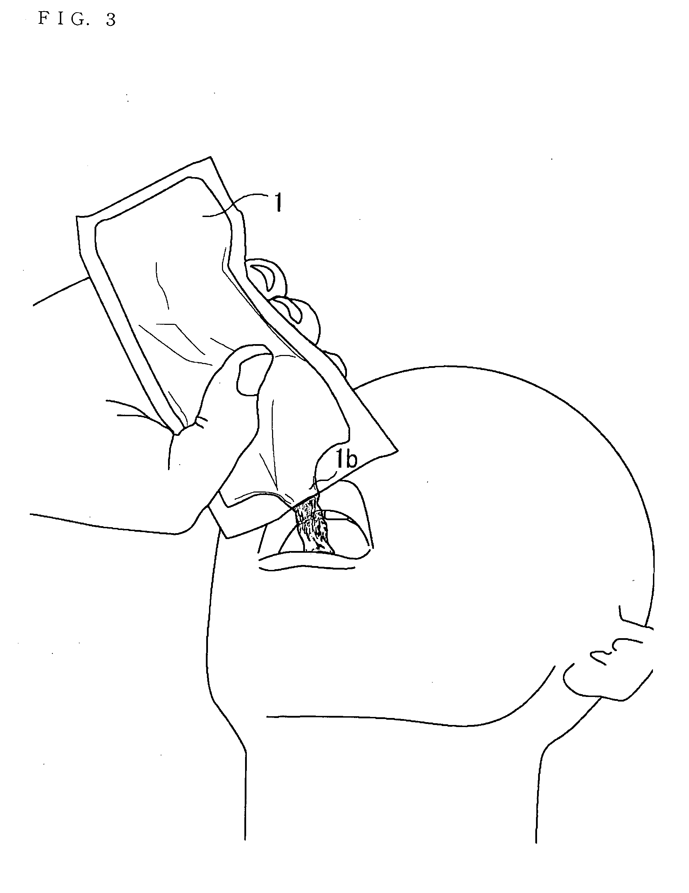

Fig. 1 is the plane view showing the case of the operation of lamination inner tube packaging, Fig. 2 is the perspective diagram of Fig. 1, and Fig. 3 is the perspective diagram of a recipe state. The laminated tube 1 has an extraction mouth 1b, and cut 1a is prepared in the edge of the laminated tube 1. The diabetic cuts the cut 1a quickly, and as shown in Fig. 3, swallows, extruding the internal supplement food.

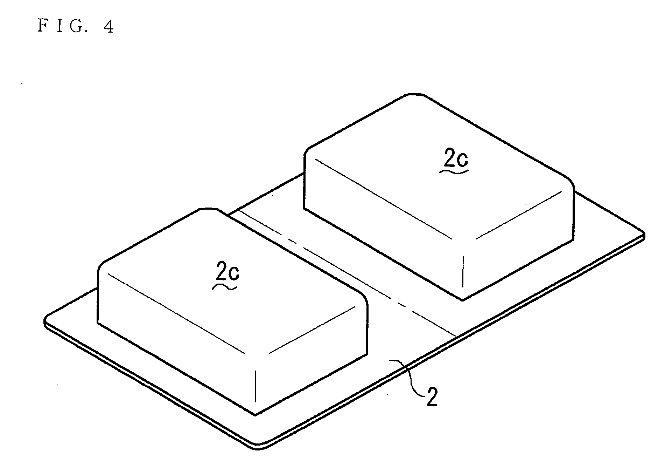

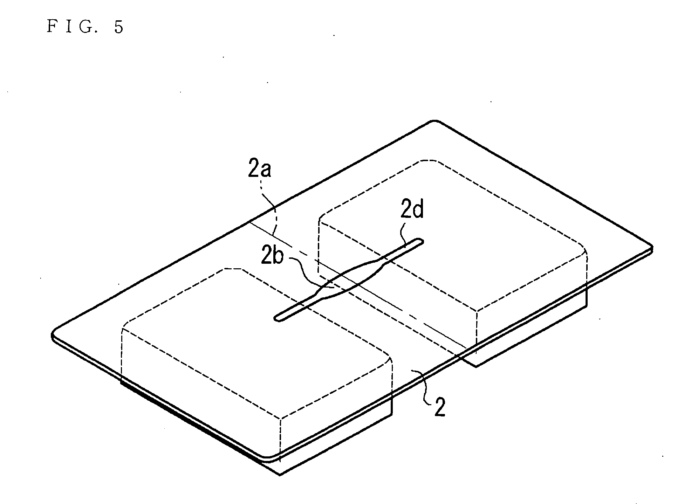

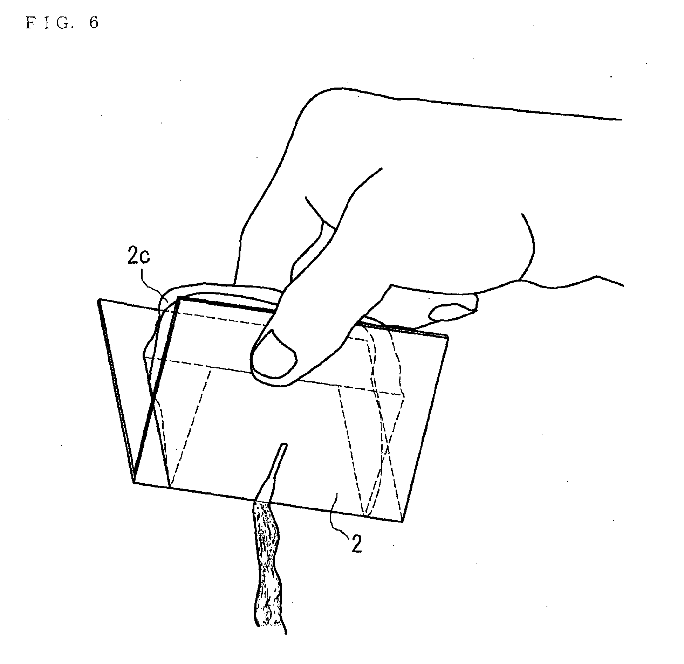

Fig. 4 is the perspective diagram showing the bending opening container 2 with which extraction mouth 2b opened only by bending operation, Fig. 5 is the perspective diagram in which showing the front side of the bending opening container 2, and Fig. 6 is the perspective diagrams in the state where carried out the opening of the bending opening container 2. In the case of the bending opening container 2, container part 2c is prepared in right and left in the state of separated on the front side. Between container parts 2c on either side is combined in slots 2d, as shown in Fig. 5. And line 2a is made on the center of container part 2c on either side, and it is easy to bend by line 2a. Who wants to take in pushes and bends an end on either side, and makes a back side project by the line 2a. Thereby, extraction mouth 2b of the center of slots 2d has been opened. And the supplement food is made to extrude and breathe out without special opening operation. In this case, since only operation which bends a container 2 from right and left is carried out, one can eat a supplement food by easy opening operation.

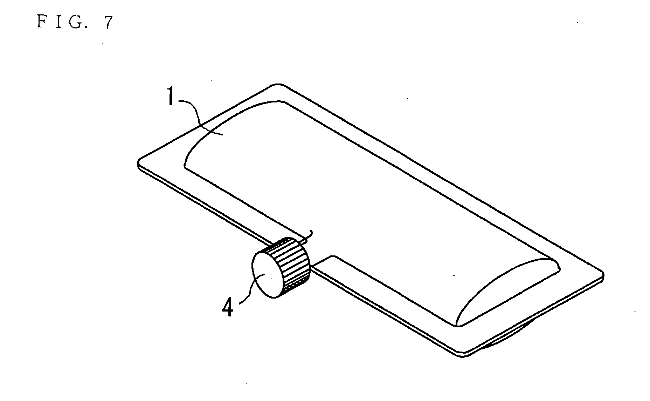

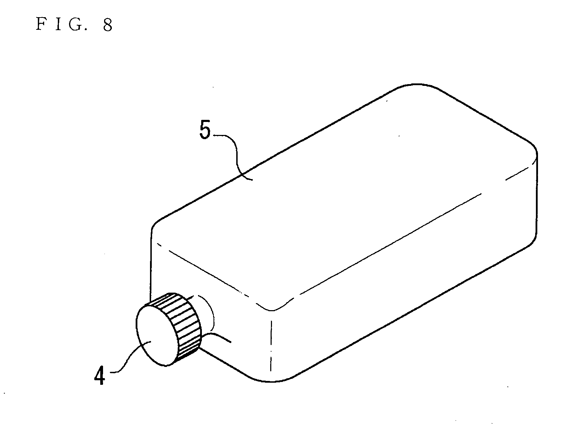

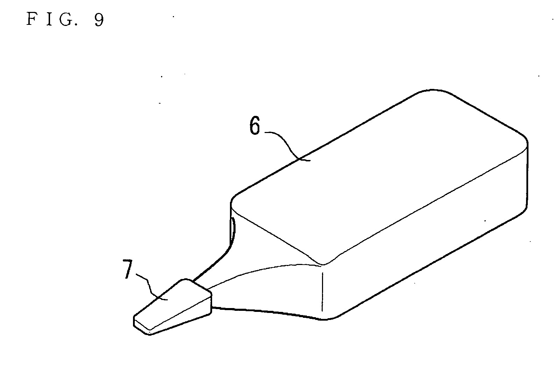

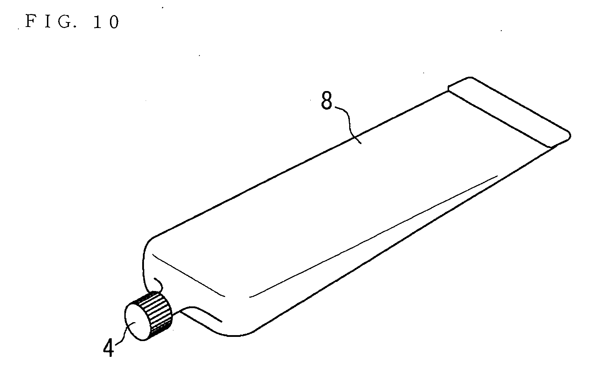

Fig. 7 is a perspective diagram of laminated container which has the screw cap 4 formed in the extraction part. Fig. 8 is a perspective diagram of the container of the mold which the small container made of a resin and the screw cap 4 combined. Fig. 9 is a perspective diagram of the small container made of a resin, and a bending section type container. Fig. 10 is a perspective diagram of aluminum foil or the tube type container made of a resin. It is necessary to turn, remove and open a screw cap 4 in the case of the operation of Fig. 7 and Fig. 8. However, not to open easily is an advantage. It is the result to cut and open by bending in the case of Fig. 9. For this reason, opening is easy. In the case of Fig. 10, it is a container with more high safety. A screw cap 4 can be used for a patient with to some extent clear consciousness, even if low blood glucose condition comes out.

Next, the change of the blood glucose level along time after taking a jelly-like supplement food is explained. Glucose was taken in with a different form. And time change of the blood glucose level was measured. It explains based on this result.

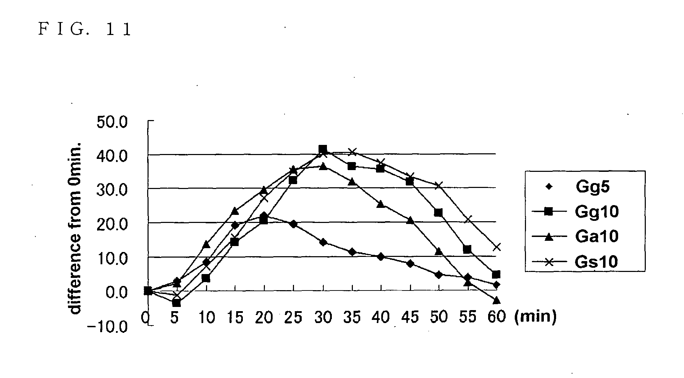

Fig. 11 is a figure showing time change of the ingestion form of glucose, and the blood glucose level.

First, four kinds of food containing glucose was created. Fixed time measurement of the change of the blood glucose level of the subjects who took in was carried out. Four kinds of food was the thing which mixed glucose 5g with the jelly, the thing which mixed glucose 10g with the jelly, the thing which melted glucose 10g with water, and 10g glucose powder.

To 6.6g of gelatinizer in prescription of Table 2, what mixed glucose 5g with the jelly adds 5g of glucose, and gels it. Glucose concentration is 43 % of the weight. In addition, a gelatinizer is a weight ratio and adds gelatin 2 and agar 0.05 to purification water 63.5. Gelatin, agar, and glucose were added and agitated in purification water. It heated for 5 minutes at 85 °C next. And it cooled and the jelly-like supplement food was obtained.

The jelly with glucose 10g is what gelled by adding glucose 10g to gelatinizer 20g of Table 2, adds 10g of glucose, and gels it, it was 33 % of the weight.

Solution containing glucose 10g with water was made from dissolving glucose 10g in water 20g. And 10g glucose powder was swallowed with one's saliva without water for washing down.

The axis of abscissas of Fig. 11 is a time-axis. Time immediately after ingestion of food was set to 0. The axis of ordinates shows the amount of change of the blood glucose level after ingestion of food. In Fig. 11, the points of lozenge showed what mixed glucose 5g with the jelly, and it was expressed with Gg5. The points of square showed what mixed glucose 10g with the jelly, and it was expressed with Gg10. The points of triangle showed what mixed glucose 10g with the jelly, and it was expressed with Ga10. The points of X showed what mixed glucose 10g with the jelly, and it was expressed with Gs10.

As shown in Fig. 11, also in which food, the rise of the blood glucose level was seen in 60 minutes of after ingestion. Either the jelly with glucose 10g and the 10g glucose powder showed some level falling after 5 minutes from ingestion, and, showed rising of blood glucose level after 10 minutes from ingestion more than immediately after ingestion. The jelly with glucose 5g showed a peak of a blood glucose level after the 20-minute of ingestion, and showed falling gently after that. The jelly with glucose 10g, solution containing glucose 10g, and 10g glucose powder showed peak of a blood glucose level after the 20-minute of ingestion, and falling gently after that. In measurement for 60 minutes, the jelly with glucose 10g and 10g glucose powder showed similar behavior of blood glucose level

In ingestion of glucose, the state where the jelly was mixed, and a powder state have the almost the same influence on the blood glucose level. For this reason, mixing glucose with a jelly provide a supplement food for low blood glucose condition recovery, which has the effect of a prompt blood glucose level rise like glucose powder and the effect of being easy to eat.

Next, the process of eating the gel-like supplement food is explained. Fig. 12 is a figure showing the process which swallows food, (a) is the figure showing the state where bolus is located on a tongue, (b) is the figure showing the state where bolus reached epiglottis, and (c) is the figure showing the state where bolus was sent into the esophagus.

Generally, in process of crunching, food is mixed with secreted saliva, and it becomes the lump (bolus) which is easy to swallow. It is sent to a throat (pharynx) from a mouth. In process swallowing a bolus, epiglottis is carrying out the important role. This epiglottis has prevented inflow to the trachea of food. It is explained by using Fig. 12. Crunching food mixes food with saliva and serves as bolus 35, it is sent to the pharynx by the tongue 31, and a tongue 31 is raised. When bolus 35 reaches the pharynx, epiglottis 33 falls, the entrance of a tracheal 32 is plugged up, and the entrance part of an esophagus 34 opens, after breathing has once stopped, bolus 35 is sent below in esophagus.

When eating food, see with eye and become aware of food, secret saliva, and bolus are made by digestion, send into the pharynx with a tongue, stop breathing, and pass onto the esophagus. A very complicated motion is made in an instant. However, this operation of a series of may become difficult by poor health etc. It becomes especially a problem that food is sent before a tracheal 32 fully closes by epiglottis 33.

Fig. 13 is a figure showing the inflow process of food near the epiglottis, (a) is the figure showing the inflow process of low viscous food, and (b) is the figure showing the inflow process of high viscous food. As shown in (a), when swallowing powder and juice, it would have a high possibility that food 38, low viscosity or high flow ability, rush into the throat, the epiglottis 33 closed inadequate, therefore it is possible food 38 to pass the epiglottis 33 and inflow into a trachea 32. The food entered into the trachea 32 makes choking and coughing by reflection and the food is discharged. For this reason, neither powder nor juice may be suitable as a supplement food for low blood glucose condition recovery. Especially, a powder type requires secretion of saliva, so it is hard to eat in some case. On the other hand, as shown in (b), the viscous gel-like food 37 goes along the surface of a throat wall because of its viscosity. And it is hard to reach epiglottis 33 quickly and contrary to one's expectations. It can be hard to be in choking. Moreover, since it is a gel-like, it is easy to swallow in less saliva.

This invention is to make easily eatable and absorptivity for glucose not to fall. As shown in Fig. 11, a gel, mixture of glucose 5 and the jelly has absorptivity equivalent to powder or a liquid. This gel is made from melting glucose in water, and adding gels gelatin and agar. This is much different with what wrapped glucose in jelly-like wafer paper, and with capsule fill with glucose.

Fig. 14 is a figure showing the absorption state of the gel-like thing containing glucose, (a) is a figure showing the absorption state of the gel-like thing of this invention, (b) is a figure showing absorption state although glucose was wrapped in jelly-like wafer paper. As shown in Fig. 14 (a), this invention is the gel-like thing 45 that is gelled glucose 43 melted in water. For this, glucose 43 is maintained by polymer with state melted in the water, and glucose 43 easily contact the absorption side 41 of a human body, and it is absorbed easily. That is, it is easy to absorb glucose dispersing to the jelly in contact with the absorption side 41. However, as shown in Fig. 14 (b), in a structure of what wrapped glucose in jelly-like wafer paper, a jelly 46 which constituted by the polymer 45 intervenes between the absorption side 41 and glucose 43. In order for glucose 43 to reach at the absorption side 41, it is necessary to pass into a jelly 46, and absorption of glucose is not performed quickly. The same is said of what put glucose in the capsule made from gelatin, the gelatin capsule needs to be melted with digestive juices etc., and absorption of glucose takes time. To meet the condition whose takes in, it is desirable for the supplement food that it is not required sufficient secretion of saliva and stomach acid, movement of stomach walls, and digestion, according to an ingestion person's condition. It is wanted attainment of the supplement food to the sugar absorption side of a human body and more easy absorption of glucose. The gel-like supplement food of this invention is gelled glucose solution, it hardly makes steam even if it supplies a throat quickly, and glucose is absorbable quickly and easily.

### Availability on industry

This invention is the composition of the supplement food for low blood glucose condition recovery. And it is useful for to supply nutrient to a person in the situation less secretion of sufficient saliva and un-sufficient stomach acid, less movement of stomach walls, and digestion are difficult.

## Claims

1. A supplement food for low blood glucose recovery at time of low blood glucose condition, comprising:
glucose as principal component,
wherein the supplement food is jelly-like food.

2. The supplement food for low blood glucose condition recovery as set forth in claim 1, wherein amount of the glucose is 10-20g in a packaging.

3. The supplement food for low blood glucose condition recovery as set forth in claim 1, wherein the glucose concentration is 20 - 65%.

4. The supplement food for low blood glucose condition recovery as set forth in claim 1, wherein content is 15-50g by a packaging.

5. The supplement food for low blood glucose condition recovery as set forth in claim 1, wherein hardness of the jelly is 10 - 100 g/cm² at 20°C.
